# EUROPÄISCHE PATENTSCHRIFT

(11) **EP 2 129 291 B1**
(45) Veröffentlichungstag und Bekanntmachung des Hinweises auf die Patenterteilung: **07.08.2013**
(21) Anmeldenummer: 08717262.3
(22) Anmeldetag: 29.02.2008
(51) Int. Cl.: A61B 5/151

(54) **STECHVORRICHTUNG FÜR DIE BLUTENTNAHME BEI MEDIZINISCHEN UNTERSUCHUNGEN**
PRICKING DEVICE FOR TAKING BLOOD FOR MEDICAL TESTS
DISPOSITIF DE PONCTION PERMETTANT D'EFFECTUER UN PRELEVEMENT SANGUIN DANS LE CADRE DE RECHERCHES MEDICALES

(30) Priorität: 05.03.2007 DE 102007011002; 24.05.2007 DE 102007024173
(43) Veröffentlichungstag der Anmeldung: 09.12.2009
(73) Patentinhaber: Gerresheimer Regensburg GmbH, 93047 Regensburg (DE)
(72) Erfinder: WESSEL, Robert, 93049 Regensburg (DE)
(74) Vertreter: Hannke, Christian
(86) Internationale Anmeldenummer: PCT/EP2008/052481
(87) Internationale Veröffentlichungsnummer: WO 2008/107379

(56) Entgegenhaltungen:
- US-A- 5 545 174
- US-A- 5 746 761
- US-A- 5 797 940

## Beschreibung

Die Erfindung betrifft eine Stechvorrichtung für die Blutentnahme bei medizinischen Untersuchungen mit einem Basiskörper, mindestens einer darin angeordneten und mit einem Spitzende herausfahrbaren Nadel mit einem die Nadel zumindest teilweise umfassenden Nadelhalteelement und einem Handbetätigungselement zum Auslösen einer Verschiebebewegung der Nadel zusammen mit dem Nadelhalteelement gegenüber dem Basiskörper gemäß dem Oberbegriff des Patentanspruches 1.

Stechvorrichtungen sind in vielfältiger Weise bekannt. Beispielsweise ist aus DE 196 17 000 C1 eine Vorrichtung mit einer Nadel bekannt, die zur Blutentnahme dient, wobei eine hohlnadelartige Kanüle mit einer angeschärften Schneidkante ausgebildet ist. Derartige Vorrichtungen sind üblicherweise nicht für den Einmal-Gebrauch vorgesehen und weisen aufgrund der Nadelform eine nicht immer schmerzfreie Anwendung auf.

Aus DE 44 43 276 A1 ist ein Blutentnahmesystem mit einem Blutaufnahmezylinder mit einem Kolben und einem auf einem Außengewinde des Blutaufnahmezylinders aufschraubbaren Nadel-Zylinderkopf mit Nadelhalterung bekannt. Ein derartiges Blutentnahmesystem ist ausdrücklich für die Wiederverwendung vorgesehen und dafür konzipiert, dass eine keimfreie erneute Anwendung dieses Systems ermöglicht wird. Demzufolge sind derartige Systeme kostenaufwändig gestaltet und bergen trotz alledem das Risiko von Infektionen. DE 297 18 679 U1 zeigt eine Lanzette für die Blutentnahme mit einem am Endbereich herausragenden Nadelspitzende. Die Nadel weist einen aus einem axialen Ende eines Basiskörpers herausragenden Endbereich mit dem Spitzende auf, das in einem rohrförmigen Ansatz - oder Verbindungsbereich eines Nadeleinstechkopfes eingebettet ist.

Der Einstechkopf und der Basiskörper sind einstückig ausgebildet. Ein axialer Druck wird auf diesen Basiskörper mittels einer nicht dargestellten Vorrichtung ausgeübt, um die Nadel bis zu einer bestimmten Tiefe in den Körper des Patienten eindringen zu lassen. Anschließend wird der Nadelendbereich aus dem Körper des Patienten von Hand herausgezogen. Derartige Vorrichtungen ermöglichen in der Regel keinen schmerzfreien Stechvorgang, da die Handhabung mittels einer zusätzlichen druckausübenden Vorrichtung lediglich das Einstechen jedoch nicht das wieder Herausnehmen der Nadel ermöglicht.

Aus US 5,5545,174 A ist eine Stechvorrichtung bekannt, bei der das Nadelhalteelement mit einem verschwenkbaren Steuerkurvenabschnitt verbunden ist. Ein Kunststofffederelement in Form eines Rastelementes dient zur Erzeugung einer Vorspannung mittels dem Handbetätigungselement, nachdem eine ausreichend grosse Kraft auf das Handbetätigungselement angewendet worden ist, wird dieser Widerstand überwunden und der verschwenkbare Steuerkurvenabschnitt wird durch das Handbetätigungselement dergestalt bewegt, dass das Nadelhalteelement in Vorwärts- und Rückwärtsrichtung verfahren wird.

Demzufolge liegt der vorliegenden Erfindung die Aufgabe zugrunde, eine Stechvorrichtung für die Blutentnahme bei medizinischen Untersuchungen zur Verfügung zu stellen, die für den Einmal-Gebrauch vorgesehen ist, einen nahezu schmerzfreien Stechvorgang ermöglicht, kostengünstig ist und auch von jedem Patienten selbst verwendet werden kann.

Diese Aufgabe wird durch eine Stechvorrichtung gemäß den Merkmalen des Patentanspruches 1 gelöst.

Ein wesentlicher Punkt der Erfindung liegt darin, dass bei einer Stechvorrichtung für die Blutentnahme bei medizinischen Untersuchungen mit einem Basiskörper, mindestens einer darin angeordneten und mit einem Spitzende herausfahrbaren Nadel mit einem die Nadel zumindest teilweise umfassenden Nadelhalteelement und einem Handbetätigungselement zum Auslösen einer Verschiebebewegung der Nadel zusammen mit dem Nadelhalteelement gegenüber dem Basiskörper in mindestens einem in dem Basiskörper angeordneten Bauteil ein verschieb- oder verschwenkbares, vorzugsweise bogenförmig oder mäanderförmig ausgebildetes Kunststofffederelement zur Erzeugung einer Vorspannung mittels dem Handbetätigungselement angeordnet ist. Dieses Kunststofffederelement ist mit einem in dem Basiskörper verschieb- oder verschwenkbaren Steuerkurvenabschnitt verbunden, wobei das Nadelhalteelement mit dem Steuerkurvenabschnitt verbunden ist und während eines Entspannungsvorganges des Kunststofffederelementes durch Abfahren des sich verschiebenden oder verschwenkenden Steuerkurvenabschnitts selbstständig in Vorwärts- und Rückwärtsrichtung verschiebbar ist.

Durch das Federbeaufschlagen des Nadelhalteelementes und damit der Nadel ist diese nicht nur auf selbstständige und einfache Weise ein- und ausfahrbar, so dass ein schneller und nahezu schmerzfreier Stechvorgang stattfindet, sondern es kann bei Verwendung des Federelementes aus Kunststoff das Bauteil und damit die gesamte Stechvorrichtung mittels des Kunsstoffspritzgussverfahrens auf einfache und kostengünstige Weise in großer Mengenanzahl hergestellt werden. Derartige Stechvorrichtungen sind für den Einmal-Gebrauch vorgesehen und können aufgrund des durch das Federelement in Verbindung mit dem Steuerkurvenabschnitt selbstständig und bzw. automatisch durchgeführten Einfahrens und Ausfahrens der Nadel von jeder Person, also auch von nicht geschulten Patienten, verwendet werden. Dies weist den Vorteil auf, dass beispielsweise Diabetiker, die eine regelmäßige Blutentnahme zur Überprüfung des Zuckerspiegels in dem Blut benötigen, sich auch selbst Blut abnehmen können und somit nicht einen Arzt oder ein Krankenhaus hierfür aufsuchen müssen.

Als Nadel ist nicht nur ein nadelförmiges Element mit rundem Querschnitt, sondern auch ein nadelförmiges Element mit rechteckförmigen Querschnitt und schneidförmiger Spitze, welches eine schnittartige Wunde ergibt, zu verstehen.

Gemäß einer bevorzugten Ausführungsform weist die Stechvorrichtung einen an dem Bauteil angeordneten, in einem Winkel zur Richtung der Verschiebebewegung verlaufenden, Spannkurvenabschnitt auf, der mit dem vorzugsweise bogenförmig ausgebildeten Kunststofffederelement verbunden ist und bei einer Verschiebebewegung des Bauteils gegenüber dem Basiskörper in seiner Verlaufsrichtung durch Entlanggleiten an einem am Basiskörper angeordneten Vorsprung veränderbar ist. Hierdurch wird durch das Verschieben des Bauteils innerhalb des Basiskörpers mittels des Handbetätigungselementes zeitgleich eine Vorspannung in dem Kunststofffederelement aufgebaut, wobei die Verschieberichtung des Handbetätigungselementes und damit des Bauteiles in einem ersten Zeitabschnitt die gleiche ist, wie die Verschieberichtung in einem zweiten Zeitabschnitt, in dem das Handbetätigungselement den Entspannungsvorgang des Kunststofffederelementes auslöst und dieses während des Nachvornegleitens und dem anschließenden Nachhintengleiten der Nadel aus und in das Gehäuse bzw. den Basiskörper entspannt wird. Alternativ kann anstatt des Vorsprungs eine vertiefte Kontur bzw. Ausnehmung an der Innenwand des Basiskörpers ausgebildet sein, in welche der Spannkurvenabschnitt, der nicht nutenförmig, sondern hervorstehend ausgebildet ist, eingreift.

Eine derartige Stechvorrichtung gemäß dieser Ausführungsform zeichnet sich dadurch aus, dass die Verschiebebewegungen des Nadelhalteelementes und des Bauteiles die gleiche Richtung aufweist, solange das Nadelhalteelement ausgefahren wird.

Alternativ können die Verschiebebewegungen des Nadelhalteelementes und des Bauteils senkrecht oder in einem Winkel zueinander verlaufen.

Der Vorsprung weist vorzugsweise eine dreieckige Grundform auf, um einen optimierten Ablauf der Gleitbewegung zwischen dem Vorsprung und dem Spannkurvenabschnitt und insbesondere während eines Vorbeigleitens eines freien Endes des Spannkurvenabschnittes an dem Vorsprung zu ermöglichen, da nach dem Vorbeigleiten des freien Endes an dem Vorsprung ein selbsttätiges Zurücklenken bzw. Zurückstellen des Spannkurvenabschnittes, der mit dem Kunststofffederelement verbunden ist, stattfinden soll, um das Kunststofffederelement zu entspannen. Dies stellt den Auslösevorgang für den Entspannungsablauf des Kunststofffederelementes dar.

Vorzugsweise ist das Bauteil zusammen mit dem Steuerkurvenabschnitt, dem Spannkurvenabschnitt und dem Kunststofffederelement einstückig ausgebildet, so dass eine kostengünstige und einfache Spritzgussproduktion dieses Kunststoffgesamtelementes möglich ist.

Gemäß einer weiteren Ausführungsform ist das Kunststofffederelement an dem Handbetätigungselement selber angeordnet, welches gegenüber dem Bauteil vorzugsweise schwenkbar gelagert ist. Das Kunststofffederelement ist gegen ein weiteres den Steuerkurvenabschnitt aufweisendes Schwenkelement durch Betätigung des Handbetätigungselementes vorspannbar, wobei das Schwenkelement gegenüber dem Bauteil schwenkbar gelagert ist. Dies erfordert ein Schwenkelement, welches einstückig oder zweistückig mit dem Bauteil verbunden sein kann.

Alternativ kann das Kunststofffederelement an dem Schwenkelement angeordnet sein, welches gegenüber dem Handbetätigungselement schwenkbar ausgebildet ist.

Das Handbetätigungselement weist einen Auslösevorgang zum Verschieben eines Haltevorsprungs des Nadelhalteelementes oder des Schwenkelementes aus seiner Arretierungsstellung auf. Hierdurch wird sichergestellt, dass erst bei einer bestimmten Schwenkstellung des Handbetätigungselementes ein Verschieben des Nadelhalteelementes und damit der Nadel ermöglicht wird. Idealerweise ist dieser Startvorgang von dem momentanen Spannungszustand der durch das sich schwenkende Handbetätigungselement vorspannenden Kunststofffeder abhängig, so dass bei Erreichen dieses Auslösevorsprunges ein Entspannungsvorgang eintritt, der ausreicht, um das Nadelhalteelement mit der für einen schmerzfreien Stechvorgang erforderlichen Geschwindigkeit aus- und anschließend wieder einfahren zu lassen.

Vorzugsweise weist das schwenkbare Handbetätigungselement und das Bauteil einen Einrastmechanismus zum Einrasten des schwenkbaren Handbetätigungselementes bei einer vorbestimmbaren Schwenkstellung und damit einer vorbestimmbaren Vorspannung des Kunststoffelementes in das Bauteil auf. Ein derartiger Einrastmechanismus soll sicherstellen, dass eine nochmalige Verwendung der erfindungsgemäßen Einmalstechhilfe nicht möglich ist.

Idealerweise findet ein Einrasten des schwenkbaren Handbetätigungselementes zeitgleich mit dem Auslösen eines Entspannungsablaufes des Kunststofffederelementes statt, sodass die Stechhilfe nicht vorgespannt werden kann und eine Einrastung stattfindet, ohne ein Auslösen des Entspannungsablaufes zu bewirken. Dies würde zu einem unbeabsichtigten Auslösen oder zu einem Vorspannungsverlust durch Kriechvorgänge des Kunststoffes führen können. In jedem Fall soll die Einrastkraft klein gegenüber der Auslösekraft sein, damit das Einrasten sozusagen automatisch bei der Auslösebewegung stattfindet, ohne dass der Benutzer dies differenzieren kann. Dieses Einrasten des Betätigungselementes ist für Stechhilfen gemäß sämtlicher Ausführungsformen vorgesehen. Die Verrastung soll dazu dienen, dass die Stechhilfe nach erfolgtem Auslösevorgang kein zweitesmal verwendet werden kann, in dem das Betätigungsteil keine zurückgerichtete Bewegung durchführt.

Bei sämtlichen Ausführungsformen können sowohl das Bauteil als auch das Handbetätigungselement als zwei getrennte Bauteile oder vorzugsweise einstückig ausgebildet sein.

Der Steuerkurvenabschnitt ist bevorzugt für sämtliche Ausführungsformen V-förmig in identischer oder ähnlicher Form in seinem Verlauf ausgebildet, so dass bei Erreichen des tiefsten Punktes des V-Verlaufes die Nadel in ihrer ausgefahrensten Position ist und anschließend gleich wieder eingefahren wird.

Der Steuerkurvenabschnitt ist in seinem Verlauf vorzugsweise als Steg bzw. Schiene ausgebildet, der beidseitig von dem Nadelhalteelement umfasst ist, um in Stegverlaufsrichtung entlangzugleiten. Alternativ kann innerhalb des Bauteiles oder des Schwenkelementes eine Nut angeordnet sein, die entsprechend komplementär ausgebildete Stege bzw. Vorsprünge des Nadelhalteelementes aufnimmt, um diese innerhalb der Nut entlang deren Verlaufsrichtung, vorzugsweise V-förmig, entlanggleiten zu lassen.

Weitere vorteilhafte Ausführungsformen ergeben sich aus den Unteransprüchen.

Vorteile und Zweckmäßigkeiten sind der nachfolgenden Beschreibung in Verbindung mit der Zeichnung zu entnehmen. Hierbei zeigen:
- Fig. 1a: eine Stechvorrichtung gemäß einer ersten Ausführungsform der Erfindung in einer perspektivischen Darstellung im zusammengebauten Zustand;
- Fig. 1b: eine Stechvorrichtung in perspektivischer Darstellung in einem auseinander gebauten Zustand;
- Fig. 2: in einer offenen und teilweise einen Querschnitt darstellenden Vorderansicht die Stechvorrichtung gemäß der ersten Ausführungsform der Erfindung;
- Fig. 3: in einer perspektivischen Draufsicht die Stechvorrichtung gemäß der ersten Ausführungsform der Erfindung;
- Fig. 4a-4c: verschiedene perspektivische Darstellungen eines in der Stechvorrichtung gemäß der ersten Ausführungsform angeordneten Bauteils in einzelnen Zusammenbauschritten;
- Fig. 5a-5e: in einer perspektivischen Darstellung die Stechvorrichtung gemäß der ersten Ausführungsform der Erfindung in verschiedenen Funktionszuständen;
- Fig. 6a: eine Stechvorrichtung gemäß einer zweiten Ausführungsform der Erfindung in perspektivischer Darstellung und im zusammengebauten Zustand;
- Fig. 6b: die Stechvorrichtung gemäß der zweiten Ausführungsform der Erfindung im auseinandergebauten Zustand in perspektivischer Darstellung;
- Fig. 7a: die Stechvorrichtung in offener und teilweiser Querschnitts-Vorderansicht gemäß der zweiten Ausführungsform der Erfindung;
- Fig. 7b: die Stechvorrichtung in offener und teilweiser Querschnitts-Vorderansicht gemäß der ersten Ausführungsform der Erfindung;
- Fig. 8a-8c: in perspektivischer Darstellung ein Bauteil zur Verwendung in einer Stechvorrichtung gemäß der zweiten Ausführungsform der Erfindung in einzelnen Zusammenbauschritten;
- Fig. 9a-9e: in einer perspektivischen Darstellung die Stechvorrichtung gemäß der zweiten Ausführungsform der Erfindung in verschiedenen Funktionszuständen;
- Fig. 10a: die Stechvorrichtung gemäß einer dritten Ausführungsform der Erfindung in perspektivischer Darstellung und im zusammengebauten Zustand;
- Fig. 10b: die Stechvorrichtung gemäß der dritten Ausführungsform der Erfindung in perspektivischer Darstellung und im auseinandergebauten Zustand;
- Fig. 11: das in der Stechvorrichtung gemäß der dritten Ausführungsform angeordnete Bauteil in perspektivischer Darstellung;
- Fig. 12: die Stechvorrichtung gemäß der dritten Ausführungsform der Erfindung in einer offenen und teilweise Querschnitts-Vorderansichtdarstellung;
- Fig. 13a-13d: die Stechvorrichtung gemäß der dritten Ausführungsform der Erfindung in einer offenen Darstellung und mit verschiedenen Funktionszuständen;
- Fig. 14: in einer offenen und teilweisen Querschnitts-Forderansicht die Stechvorrichtung gemäß einer vierten Ausführungsform der Erfindung;
- Fig. 15: in einer perspektivischen Darstellung ein Bauteil der Stechvorrichtung gemäß der vierten Ausführungsform der Erfindung; und
- Fig. 16a-16d: in einer perspektivischen teilweise offenen Darstellung die Stechvorrichtung gemäß der vierten Ausführungsform der Erfindung in verschiedenen Funktionszuständen.

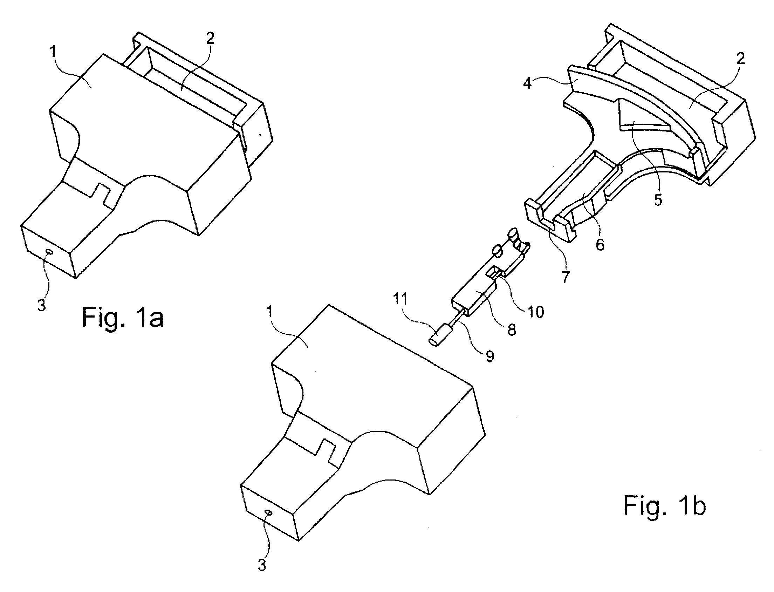

In Fig. 1a wird in einer perspektivischen Darstellung eine Stechvorrichtung gemäß einer ersten Ausführungsform der Erfindung gezeigt. Die Stechvorrichtung umfasst einen Basiskörper 1, welcher als Gehäuse ausgebildet ist und ein darin angeordnetes Bauteil 2, welches gegenüber dem Basiskörper 1 verschiebbar ist. Aus einem unteren Ende 3 des Basiskörpers 1 kann eine Nadel austreten.

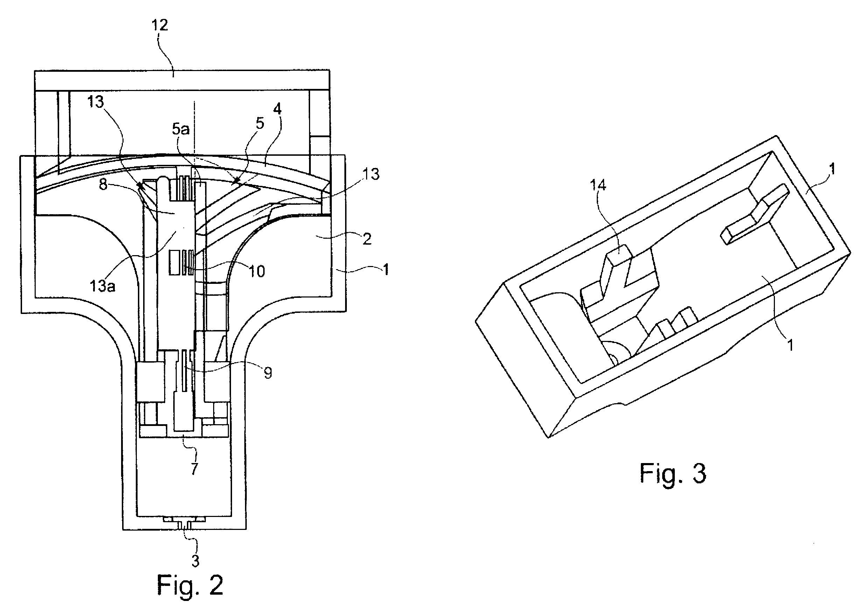

In Fig. 1 b ist in einer perspektivischen Darstellung die Stechvorrichtung gemäß der ersten Ausführungsform der Erfindung in einem auseinandergebauten Zustand wiedergegeben. Das Bauteil 2 ist aus dem Basiskörper 1 herausgenommen und weist eine Kunststofffeder 4 bzw. ein Kunststofffederelement 4 auf, welches bogenförmig ausgebildet ist und mittels einem Spannkurvenabschnitt 5, der an dem Bauteil 2 in einem Winkel 5a, wie es aus Fig. 2 hervorgeht, zu der Verschiebebewegung einer Nadel angeordnet ist, mit diesem verbunden ist.

Innerhalb einer in Längsrichtung des Basiskörpers 1 und des Bauteiles 2 ausgerichteten Ausnehmung 6 mit einer unteren Öffnung 7 ist ein Nadelhalteelement 8 angeordnet, in welchem wiederum eine Nadel 10 mit einem dazugehörigen Spitzende 9, welches durch eine Schutzkappe 11 verschlossen ist, umfasst. Das Nadelhalteelement 8 kann innerhalb der Führung 6 vor- und zurückgeschoben werden.

Die Schutzkappe 11 dient als Sterilschutz für die Nadel und wird während des Stechvorganges durchstochen. Sie besteht aus einem Elastomer.

In Fig. 2 ist in einer offenen und teilweisen Querschnitts-Vorderansicht die Stechvorrichtung gemäß der ersten Ausführungsform der Erfindung gezeigt.

Dieser Darstellung ist deutlich zu entnehmen, dass, sobald ein Handbetätigungselement 12 von oben nach unten betätigt bzw. verschoben wird, eine Verschiebung des gesamten Bauteiles 2 nach unten erfolgt.

Während eines derartigen Nachuntenverschiebens wird das Nadelhalteelement 8 mit der Nadel bzw. der Lanzette 10 ebenso verschoben in Abhängigkeit davon, wie der Kurvenverlauf eines Steuerkurvenabschnittes 13, der vorzugsweise V-förmig ausgebildet ist und einen Tiefpunkt 13a aufweist, gestaltet ist.

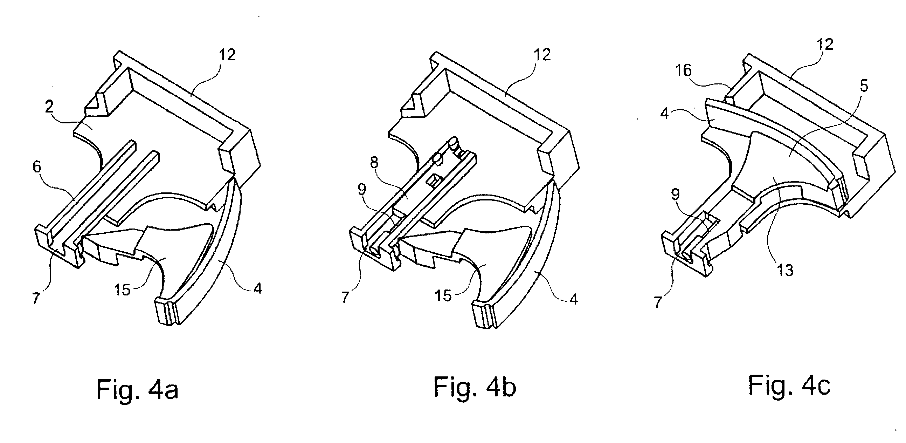

Um den Spannkurvenabschnitt 5, der mit dem Winkel 5a gegenüber einer Verschiebebewegungsrichtung bzw. einer Längsachse des Basiskörpers ausgerichtet ist und vorzugsweise leicht bogenförmig ausgebildet ist, wobei er stegartig bzw. schienenartig ausgebildet ist, in seiner Ausrichtung während der Verschiebebewegung des Handbetätigungselementes und damit des Bauteiles 2 zu verändern, weist der Basiskörper 1 einen Vorsprung 14 auf, wie er in der perspektivischen Draufsicht gemäß Fig. 3 wiedergegeben ist. Dieser basiskörperfeste Vorsprung 14 bewirkt ein Auslenken bzw. eine Änderung der Verlaufsrichtung des Spannkurvenabschnittes 5 während der Auf- und Abwärtsbewegung des Bauteiles 2, wobei sowohl der Spannkurvenabschnitt als auch der Steuerkurvenabschnitt und das Kunststofffederelement 4 an einem am Bauteil 2 schwenkbar aufgehängten Schwenkbauteil 15, wie es aus den Fig. 4a, 4b, 4c hervorgeht, angeordnet ist. Dieses Schwenkbauteil ist mit dem Rest des Bauteiles 2 vorzugsweise einstückig oder alternativ zweistückig verbunden.

In Fig. 4a - 4c wird das Bauteil 2 in einzelnen Zusammenbauschritten wiedergegeben.

In Fig. 4a setzt sich das Bauteil 2 sowohl aus der Führungsaussparung 6 mit der unterseitigen Öffnung 7 als auch aus dem Schwenkbauteil 15, welches an seiner unteren Seite vorzugsweise einstückig gelenkig mit dem restlichen Bauteil 2 verbunden ist, und dem Kunststofffederelement 4 zusammen.

Das Handbetätigungselement ist vorzugsweise einstückig mit dem Bauteil 2 verbunden.

In einem ersten Zusammenbauschritt wird der vorzugsweise einstückig gegossene Körper des Bauteiles 2 zusammen mit dem Schwenkbauteil 15 angeordnet und anschließend wird, wie in Fig. 4b wiedergegeben, das Nadelhalteelement 8 mit dem Nadelspitzende 9 in die Führungsaussparung 6 eingesetzt.

Anschließend findet in einem dritten Zusammenbauschritt gemäß Fig. 4c die richtige Platzierung des Kunststofffederelementes 4, welches an einem Ende 16 des Bauteiles 2 zwar vorbeigleiten kann, jedoch von diesem aufgrund der schrägen Ausbildung des Endes 16 während einer Vorspannungs- und Entspannungsphase geführt wird, statt.

Das Schwenkbauteil 15 kann gegenüber dem restlichen Bauteil 2 mittels eines Filmscharniers vorzugsweise auf Kunststoffbasis verbunden sein. Die Spann- und Steuerkurvenabschnitte können nicht nur an dem Schwenkbauteil 15, sondern auch an dem Basiskörper 1 und/oder dem Nadelhalteelement 8 ausgebildet sein, wobei die jeweils zugehörigen Nocken und Nutenführungen entsprechend den damit zusammenwirkenden Bauteilen komplementär ausgebildet sind. Alternativ zu einem Kunststofffederelement kann auch eine Stahlfeder verwendet werden, wobei eine derartige Stahlfeder bereits vorgespannt in einer derartigen Stechvorrichtung montiert werden kann. Dies ermöglicht das Entfallen eines Vorspannweges und des Spannkurvenabschnittes.

In Fig. 4c sind deutlich der Spannkurvenabschnitt 5 und der Steuerkurvenabschnitt 13 zu sehen, entlang welcher zum Einen ein basiskörperfester Vorsprung 14 und zum Anderen das Nadelhalteelement 8, welches in den Steuerkurvenabschnitt 13, der ebenso steg- oder schienenartig ausgebildet ist, ober- und unterseitig eingreift, gleitet.

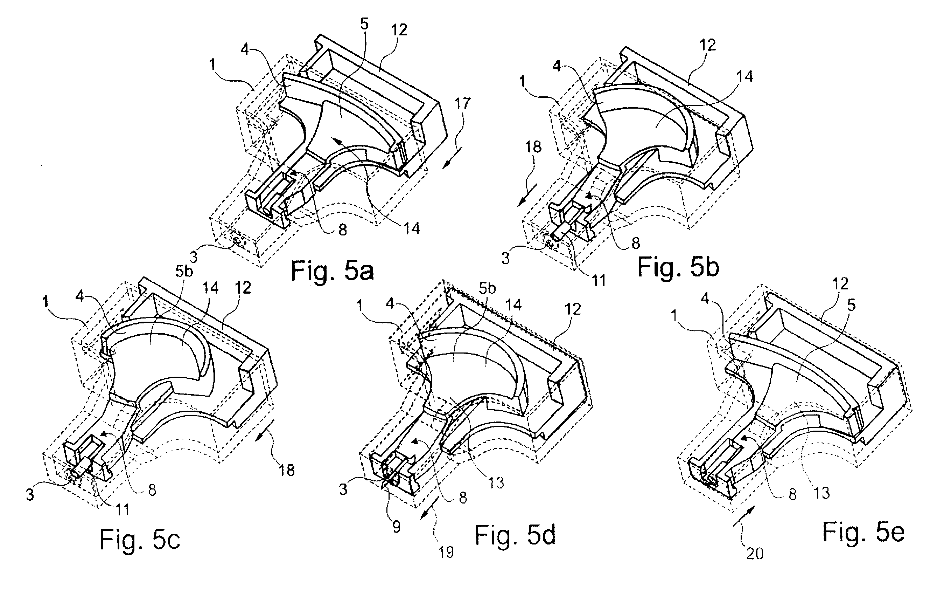

In Fig. 5a - 5e sind verschiedene Funktionszustände der Stechvorrichtung gemäß der ersten Ausführungsform der Erfindung in perspektivischer Darstellung wiedergegeben.

In Fig. 5a ist die Stechvorrichtung vor deren Gebrauch, also in unbenutztem Zustand dargestellt. Sie umfasst den Basiskörper 1 mit dem darin angeordneten Bauteil 2 und dem unterseitigen Nadelausgang 3 sowie dem Handbetätigungselement 12.

Der Spannkurvenabschnitt 5 ist gegenüber dem basiskörperfesten Vorsprung 14, der vorzugsweise eine dreieckige Grundform aufweist, gleitend gelagert.

Gemäß dem Pfeil 17 kann durch Drücken des Handbetätigungselementes 12 von oben das gesamte Bauteil 2 in dem Basiskörper 1 hineingeschoben werden.

In Fig. 5b ist der Funktionszustand des Vorspannens des Kunststofffederelementes 4 dargestellt. Durch ein Nachuntendrücken des Handbetätigungselementes 12 in dem Basiskörper 1 hinein findet ein Entlanggleiten des Spannkurvenabschnittes 5 an dem Vorsprung 14 statt, wodurch sich durch Verschwenken des Schwenkteiles 15 das Kunststofffederelement 4 vorspannt, indem es nach links gedrückt wird. Dies wird durch eine verstärkte Bogenausbildung des Kunststofffederelementes 4 sowohl in Fig. 5b als auch in Fig. 5c wiedergegeben.

Eine derartige Vorspannbewegung durch ein Verschieben des Bauteiles 2 in Richtung des unteren Endes des Basiskörpers 1, wie es durch den Pfeil 18 wiedergegeben wird, bewirkt zudem, dass das Nadelhalteelement 8 mit zwei hervorstehenden Nockenelementen bzw. einer in dem Nadelhalteelement angeordneten Nut entlang eines Stegs bzw. einer Schiene des Steuerkurvenabschnittes 13 von links nach rechts verläuft. Hierbei durchläuft das Nadelhalteelement den Tiefpunkt 13a des Steuerkurvenabschnittes 13, jedoch zu einem Zeitpunkt, zu dem das Bauelement 2 noch nicht vollständig bis zum Anschlag in dem Basiskörper 1 hineingeschoben worden ist. Hierdurch ergibt sich, dass zwar die Nadelspitze 9 kurzzeitig nach unten und anschließend wieder nach oben aufgrund des V-förmigen Verlaufes des Steuerkurvenabschnittes geschoben wird, jedoch noch nicht aus dem unteren Ende 3 des Basiskörpers 1 heraustritt.

Sobald - wie in Fig. 5c als Startzeit des Entspannungsvorganges wiedergegeben - der Vorsprung 14, der nockenartig ausgebildet ist, ein freies Ende 5b des Spannkurvenabschnittes 5 erreicht hat, wird der Spannkurvenabschnitt 5 durch den Vorsprung bzw. Nocken 14 freigegeben, woraufhin ein Entspannungsvorgang des Kurvenfederelementes ausgelöst wird. Dies bewirkt ein Zurückverschwenken des Schwenkteiles 15 in seinen ursprünglichen Zustand und ein Entspannen des Kunststofffederelementes 4.

Während eines derartigen Entspannungsvorganges durchläuft das Nadelhalteelement 8 noch einmal, diesmal jedoch in umgekehrter Abfolge - den Steuerkurvenabschnitt 13 - woraufhin während des Durchlaufens des untersten Punktes 13a des Steuerkurvenabschnittes 13 die Nadel 10 zusammen mit dem Spitzende 9 und das Nadelhalteelement 8 kurzzeitig nach vorne bewegt werden. Dies geschieht in einem Ausmaß, dass das Spitzende 9 kurzzeitig aus der Öffnung 3 des Basiskörpers 1 herausbewegt wird und anschließend gleich wieder zurückgeschoben wird, da das Nadelhalteelement 8 nun den zweiten Abschnitt des Steuerkurvenabschnittes 13, nämlich den linken Anteil, durchläuft. Eine derartige Stechbewegung ist also federgetrieben und linear im Bewegungsablauf, woraus sich eine nahezu benutzerunabhängige Stechbewegung und damit eine hinsichtlich der verursachenden Schmerzen nahezu neutrale Bewegung der Nadel ergeben. Die lineare Stechbewegung wird durch den Pfeil 19 wiedergegeben.

In Fig. 5e wird die Stechvorrichtung nach ihrem Gebrauch dargestellt. In diesem Funktionszustand ist die Nadelspitze wieder in den Basiskörper 1, ebenso wie das Nadelhalteelement 8, hineingefahren, wie es durch den Pfeil 20 wiedergegeben wird.

Das Bauteil 2 hingegen bleibt innerhalb des Basiskörpers 1 angeordnet und bewegt sich nicht mehr zurück. Nach einem derartigen Einmal-Gebrauch wird eine derartige Stechvorrichtung nicht zu einer weiteren Verwendung vorgesehen.

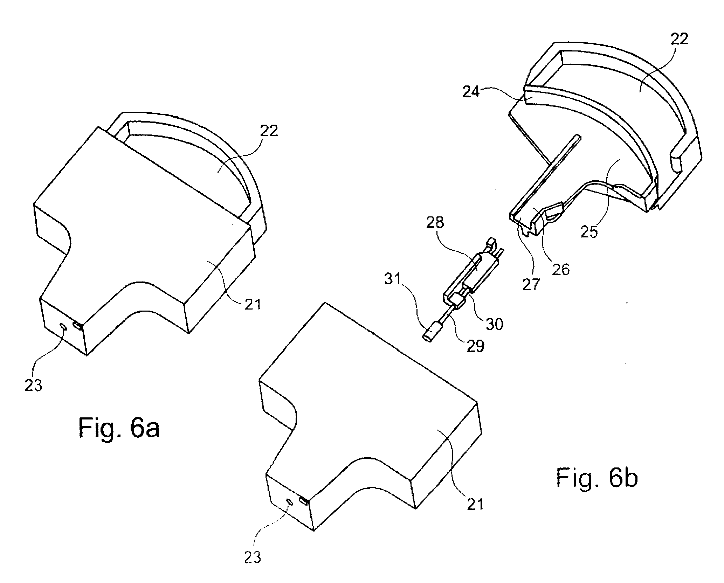

In Fig. 6a ist in einer perspektivischen Darstellung eine Stechvorrichtung gemäß einer zweiten Ausführungsform der Erfindung gezeigt. Diese Stechvorrichtung umfasst wiederum einen Basiskörper 21 zusammen mit einem Bauteil 22 und einem unterseitig offenen Ende 23, welches lochartig ausgebildet sein kann.

In Fig. 6b ist die Stechvorrichtung gemäß der zweiten Ausführungsform der Erfindung in perspektivischer Darstellung auseinandergebaut wiedergegeben. Das Bauteil 22 umfasst wiederum das Kunststofffederelement 24 und den Spannkurvenabschnitt 25. Es ist ebenso die Führungsaussparung 26 mit einem unteren Ende 27 zur Aufnahme des Nadelhalteelementes 28 vorhanden.

Das Nadelhalteelement 28 umfasst wiederum eine Nadel 30 mit einem Spitzende 29, welches mittels einer Schutzkappe 31 abgedeckt ist.

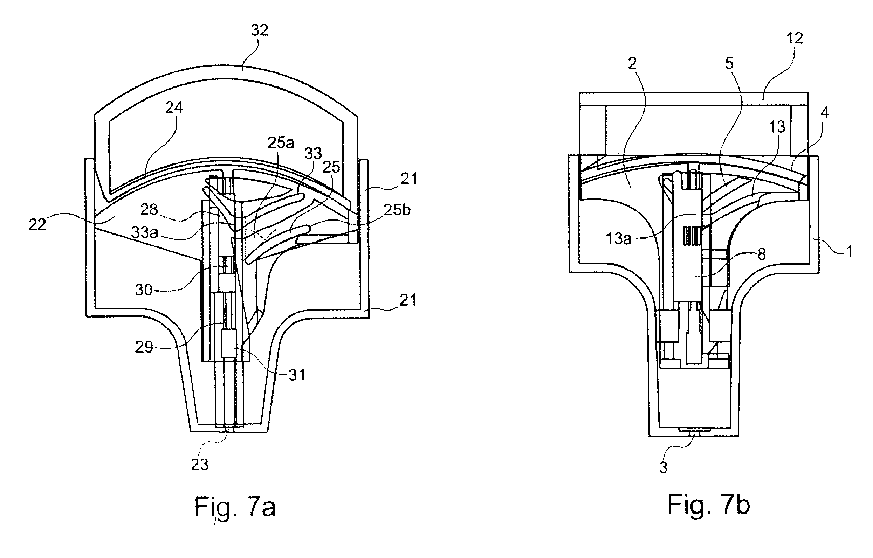

In Fig. 7a und 7b sind die Stechvorrichtungen gemäß der zweiten und der ersten Ausführungsform in einer offenen und teilweise Querschnitts-Vorderansicht zu vergleichenden Zwecken wiedergegeben.

Die in Fig. 7a wiedergegebene Stechvorrichtung gemäß der zweiten Ausführungsform der Erfindung zeigt das Bauteil 22 mit dem Kunststofffederelement 24 und dem Spannkurvenabschnitt 25, welcher wiederum in einem Winkel 25a zur Verschieberichtung eines Handbetätigungselementes 32 und damit des Bauteiles 22 und auch zu der Verschiebebewegung der Nadel 30 zusammen mit dem Nadelhalteelement 28 angeordnet ist. Wiederum kann der Spannkurvenabschnitt 25 leicht bogenförmig ausgebildet sein und aus unelastischem oder elastischem Material hinsichtlich seiner Bogenverformbarkeit bestehen. Ebenso kann der Spannkurvenabschnitt 25 sowohl nutenförmig mit entsprechend komplementär ausgebildeten eingreifenden/m Nocken, die bzw. der an dem Basiskörper 1 angeordnet sind/ist, oder als Steg bzw. Schiene, an welche(r) ein Nocken, der basiskörperfest ist, entlanggleiten kann, ausgebildet sein.

Der Spannkurvenabschnitt 25 umfasst wiederum ein freies Ende 25b.

Ein Steuerkurvenabschnitt 33 ist wiederum V-förmig in identischer oder ähnlicher Form ausgebildet und weist einen unteren tiefsten Steuerkurvenabschnittspunkt 33a auf.

Im Gegensatz zu der in Fig. 7b wiedergegebenen Stechvorrichtung gemäß der ersten Ausführungsform wird bei der in Fig. 7a wiedergegebenen Stechvorrichtung gemäß der zweiten Ausführungsform die Rückbewegung des Schwenkteiles 15, welches eine Federauslenkung zeitgleich mit Erreichen der Endstellung des Bauteils 22 und des Betätigungselementes 32 bewirkt. Dies wird durch einen festen Anschlag innerhalb des Gehäuses erreicht.

Demgegenüber wird bei der Stechvorrichtung gemäß der ersten Ausführungsform der Erfindung in Fig. 7b eine Rückbewegung des schwenkbaren Bauteils bereits vor Erreichen des Bauteiles 2 bzw. des Handbetätigungselementes 12 in seiner Endstellung, also vor dem kompletten Einschieben des Bauteiles 2 in den Basiskörper erhalten.

Dadurch überlagert sich der Restweg des Handbetätigungselementes 12 bzw. des Bauteiles 2 innerhalb des Basiskörpers 1 mit der Schwenkbewegung des Schwenkteiles 15, die den Stechhub mitausführt. Hierbei ist der gehäusefeste Vorsprung 14 derart gestaltet, dass zum Einen der Federdruck die Linearverschiebung des Bauteiles 2 noch unterstützt und zum Anderen das schwenkbare Teil 15 erst dann die maximale Lanzettenaustrittsweite bzw. Spitzendeaustrittsweite erreicht, wenn das Bauteil 2 seinen Endanschlag innerhalb des Basiskörpers erreicht hat. Dies ist wichtig für die Wiederholgenauigkeit bei der Stechtiefe.

Vorteilhaft weist die Stechvorrichtung gemäß der ersten Ausführungsform der Erfindung eine geringere Gesamtbaugröße auf, da das schwenkbare Teil 15 einen kleineren Winkel überstreichen muss und somit auch robuster ausgestaltbar ist, da das Kunststofffederelement weniger stark gespannt werden muss.

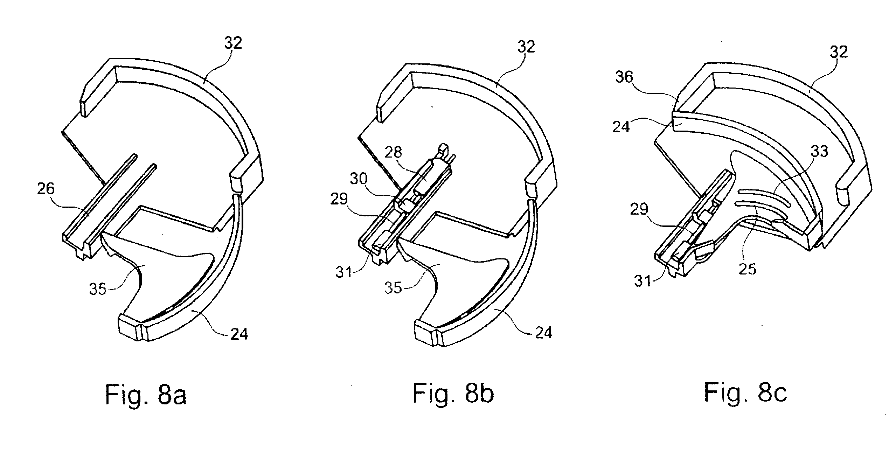

In Fig. 8a - 8c wird die Stechvorrichtung gemäß der zweiten Ausführungsform der Erfindung in perspektivischer Darstellung mit verschiedenen Zusammenbauschritten wiedergegeben. Wiederum weist das Bauteil 22 ein schwenkbares Teil 35 auf, welches unterseitig schwenkbar, vorzugsweise mittels eines Filmscharniers, aufgehängt ist.

Anschließend wird in einem zweiten Schritt gemäß Fig. 8b das Nadelhalteelement 28 eingesetzt.

In einem dritten Schritt findet das Zusammenschwenken des Schwenkteils 35 und des restliches Bauteiles 22 derart statt, dass das Kunststofffederelement 24 an einer Unterseite des Bauteiles 22 vorbeigleiten kann, wobei die Unterseite 36 leicht abgeschrägt zum reibungsarmen Vorbeigleiten ausgebildet ist.

Der Steuerkurvenabschnitt 33 ist wiederum V-förmig ausgebildet. Der Spannkurvenabschnitt 25 liegt unterhalb des Steuerkurvenabschnittes 33.

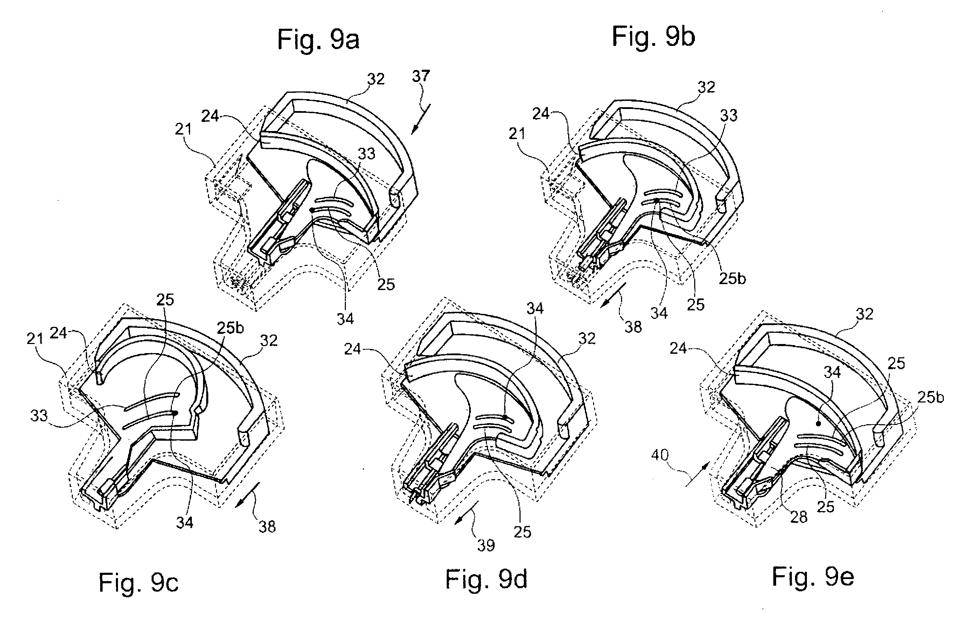

In Fig. 9a - 9e ist die Stechvorrichtung gemäß der zweiten Ausführungsform der Erfindung in perspektivischen Darstellungen während ihres Funktionsablaufes wiedergegeben.

In Fig. 9a wird der Funktionszustand vor Gebrauch der Stechvorrichtung gezeigt. Das Bauteil 22 befindet sich zusammen mit dem Handbetätigungshebel 32 noch rückseitig etwas außerhalb des Basiskörpers 21 und wird durch Druckbeaufschlagung von Hand, wie es durch den Pfeil 37 wiedergegeben wird, in den Basiskörper 21 hineingeschoben.

In Fig. 9b wird in einer perspektivischen Darstellung das Hineinschieben des Bauelementes 22 in den Basiskörper 21 wiedergegeben, wie es durch den Pfeil 38 dargestellt ist. Während dieses Hineinschiebens findet eine Vorspannung des Kunststofffederelementes 24 durch Verschwenken des Schwenkteiles 35 nach links statt. Dies geschieht durch ein Entlanggleiten des Spannkurvenabschnittes 25 an dem basiskörperfesten Vorsprung 34, der wiederum vorzugsweise dreieckförmig ausgebildet sein kann.

Bei Erreichen eines freien Endes 25b des Spannkurvenabschnittes 25 wird, wie in Fig. 9c dargestellt, die auf ihre Maximalspannung vorgespannte Kunststofffeder 24 schlagartig entlastet, so dass ein Entspannungsvorgang des Kunststofffederelementes 24 eingeleitet wird. Dies findet dadurch statt, dass der Vorsprung 34 den Spannkurvenabschnitt 25 freigibt, indem er das freie Ende 25b des Spannkurvenabschnittes 25 überschreitet.

Während des Entspannungsvorganges wird das Nadelhalteelement 28 innerhalb der Führungsaussparung 26 nach vorne geschoben, da es den V-förmigen Steuerkurvenabschnitt innerhalb kürzester Zeit von rechts nach links durchläuft und kurzzeitig des niedrigsten Punktes 33a des Steuerkurvenabschnittes 33 erreicht. Während dieses Erreichens tritt das Ende 29 der Nadel 30 aus der unterseitigen Öffnung 23 des Basiskörpers 21 kurzzeitig aus und wird anschließend sofort wieder in den Basiskörper hineingezogen, wie es durch den Pfeil 40 in Fig. 9e wiedergegeben ist. Fig. 9e zeigt hierbei den Zustand der gesamten Stechvorrichtung gemäß der zweiten Ausführungsform der Erfindung nach ihrem Gebrauch.

Selbstverständlich durchläuft das Nadelhalteelement mit einer darin angeordneten Nut oder zwei darauf angeordneten Vorsprüngen den Steuerkurvenabschnitt, der steg- oder schienenartig ausgebildet ist, bereits von links nach rechts während des Nachuntenschiebens des Betätigungselementes 32 ein erstes Mal, bevor es ein zweites Mal den Steuerkurvenabschnitt in umgekehrter Richtung durchläuft, um bei diesem zweiten Mal die Nadel im Gegensatz zu dem ersten Mal aus dem Basiskörper herausschieben zu lassen.

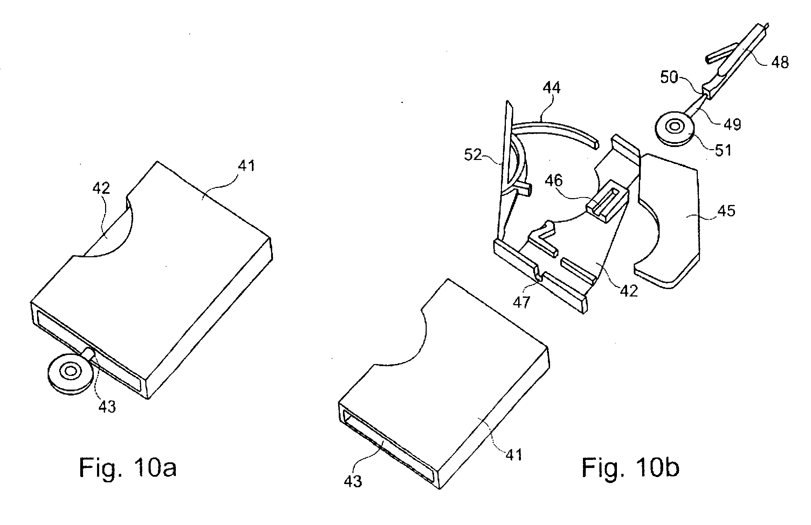

In Fig. 10a ist in einer perspektivischen Darstellung eine Stechvorrichtung gemäß einer dritten Ausführungsform der Erfindung gezeigt. Diese Stechvorrichtung weist wiederum einen Basiskörper 41 mit einem darin angeordneten Bauteil 42 und einer unterseitigen Öffnung 43 auf.

In Fig. 10b ist die Stechvorrichtung gemäß der dritten Ausführungsform in auseinandergebautem Zustand wiedergegeben. Diese Stechvorrichtung weist an ihrem Bauteil vorzugsweise einstückig angeordnete mehrere Element auf, welche das Kunststofffederelement 44, ein schwenkbares Teil 45, welches oberseitig an dem Bauteil schwenkbar angeordnet ist und unterseitig einen Steuerkurvenabschnitt aufweist und ein Betätigungselement 52 umfassen.

Wiederum ist eine Führungsaussparung 46 in dem Bauteil 42 angeordnet, um ein Nadelhalteelement 48 mit einer darin angeordneten Nadel 50, die ein Spitzende 49 mit darauf angebrachter Schutzkappe 51 aufweist, anzuordnen.

Die Schutzkappe 51 kann vor der Betätigung der Stechhilfe in einem separaten Bedienschritt abgedreht werden und dient als Sterilschutz.

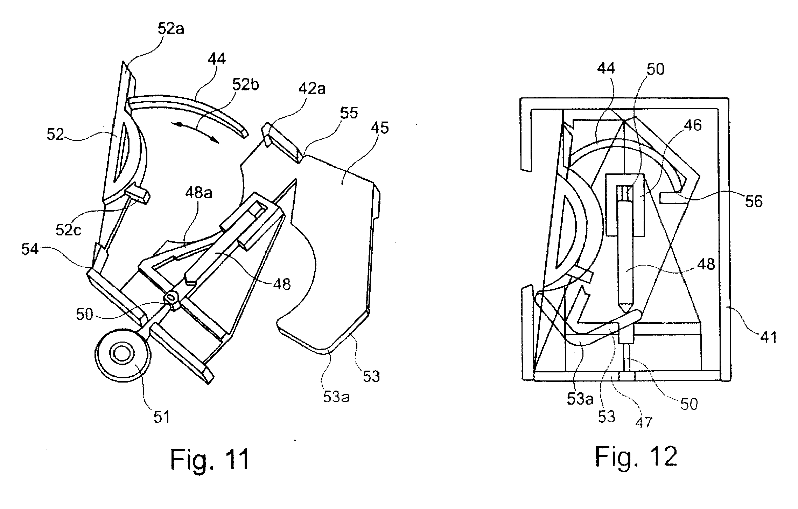

In Fig. 11 ist das Bauteil 42 gemäß der Stechvorrichtung nach der dritten Ausführungsform nochmals wiedergegeben. Das Bauteil 42 umfasst das schwenkbare Teil 45, welches oberseitig mittels eines Filmscharniers 55 schwenkbar angeordnet ist. Unterseitig ist das schwenkbare Teil mit einem Steuerkurvenabschnitt 53, der wiederum V-förmig in identischer oder ähnlicher Form ausgebildet ist und einen tiefsten Punkt 53a aufweist, angeordnet.

Das Kunststofffederelement 44 ist zusammen mit dem Handbetätigungshebel 52, der schwenkbar an einem unteren Punkt 54 mittels eines Filmscharniers an dem restlichen Bauteil 42 angeordnet ist, gegenüber dem restlichen Bauteil 42 schwenkbar, wie es durch den Doppelpfeil 52 wiedergegeben wird.

Das schwenkbare Handbetätigungselement 52 kann mit seinem oberen Ende 52a in eine entsprechend komplementär ausgebildete Eckkante 42a des Bauteiles 42 einrasten, sobald das Handbetätigungselement entsprechend weit in den Basiskörper hineingeschwenkt worden ist.

In Fig. 12 wird wiederum in einer offenen und teilweise querschnittsartigen Vorderansicht die Stechvorrichtung gemäß der dritten Ausführungsform der Erfindung gezeigt. Dieser Darstellung ist deutlich zu entnehmen, dass das Kunststofffederelement 44 gegenüber einen Anschlag 56 des schwenkbaren Teiles 45 lagerbar ist, um bei weiterer Betätigung des Handbetätigungselementes 52 eine Auslösung des Nadelhalteelementes zu bewirken.

Das Nadelhalteelement 48 ist mittels einer Nut den Steuerkurvenabschnitt 53 ober- und unterseitig umfassend entlang dieses Abschnittes gleitbar, sofern sich der Steuerkurvenabschnitt 53, der schienen- oder stegartig ausgebildet ist, von links nach rechts bewegt.

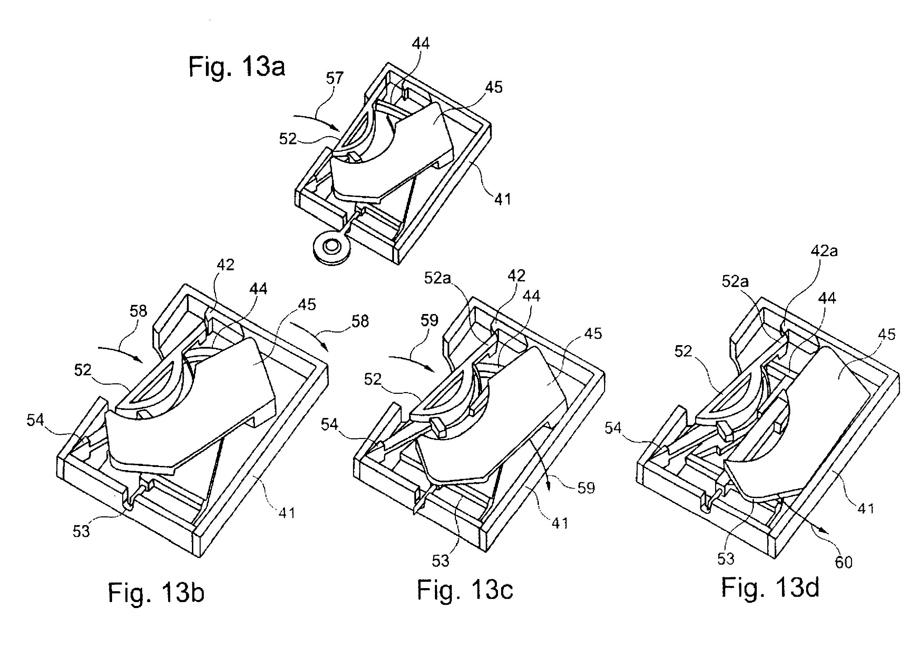

In Fig. 13a - d sind wiederum verschiedene Funktionszustände der Stechvorrichtung gemäß der dritten Ausführungsform der Erfindung in einer perspektivischen Darstellung wiedergegeben. In Fig. 13a wird die Stechvorrichtung vor deren Gebrauch dargestellt. Zu Anfang wird das Handbetätigungselement 52, welches seitlich angeordnet ist, nach rechts verschwenkt, wie es durch den Pfeil 57 wiedergegeben ist.

Durch ein derartiges Nachrechstverschwenken des Handbetätigungselementes 52 findet - wie in Fig. 13b wiedergegeben - ein Vorspannungsvorgang des Kunststofffederelementes 44 statt, in dem dieses mit seinem rechten hier nicht näher dargestellten Ende gegen den in Fig. 12 wiedergegebenen Anschlag 56 des schwenkbaren Teiles 45 gedrückt wird. Hierdurch findet eine verstärkte Bogenform des Kunststofffederelementes 44 und somit der Aufbau einer Vorspannung statt. Dies wird durch den Pfeil 58 wiedergegeben.

In Fig. 13c findet nun durch das Betätigen eines Arretierungshebels 48a, der in Fig. 11 näher dargestellt und mit dem Nadelhalteelement 48 verbunden ist, mittels eines Vorsprunges 59c des Handbetätigungselementes 52 eine Entsperrung des Arretierungszustandes des Nadelhalteelementes 48 statt, wodurch sich die vorgespannte Kunststofffeder 44 schlagartig entspannen kann und das schwenkbare Teil 45 schlagartig nach rechts geschwenkt wird, wie es durch die Pfeile 59 und 60 in den Figuren 13c und 13d wiedergegeben wird.

Ein derartiges Verschwenken des schwenkbaren Teiles 45 insbesondere seines unteren Endes ermöglicht, dass das Nadelhalteelement 48 entlang dem Steuerkurvenabschnitt 53, welcher am unteren Ende des schwenkbaren Teiles 45 angeordnet ist, von rechts nach links verfahren werden kann, woraufhin die Nadelspitze 49 kurzzeitig aus dem unteren Ende 43 bzw. 47 des Basiskörpers bzw. des Bauteiles 42 austritt und sofort wieder nach Überwinden des tiefsten Punktes 53a des Steuerkurvenabschnittes 53 in die Stechvorrichtung hineingezogen wird. Hierdurch findet ein benutzerunabhängiger, schneller und einfacher Stechvorgang statt.

Eine derartige Schwenkbewegung des schwenkbaren Teiles 45 in kontrollierter Weise ist, wenn das Kunststofffederelement 44 fertig vorgespannt ist, zeitgleich mit einer Einrastung des oberen Endes 52a des Handbetätigungselementes 52 in den Eckabschnitt 42a des Bauteiles 42 möglich, wie es durch die Darstellung gemäß Fig. 13c wiedergegeben wird.

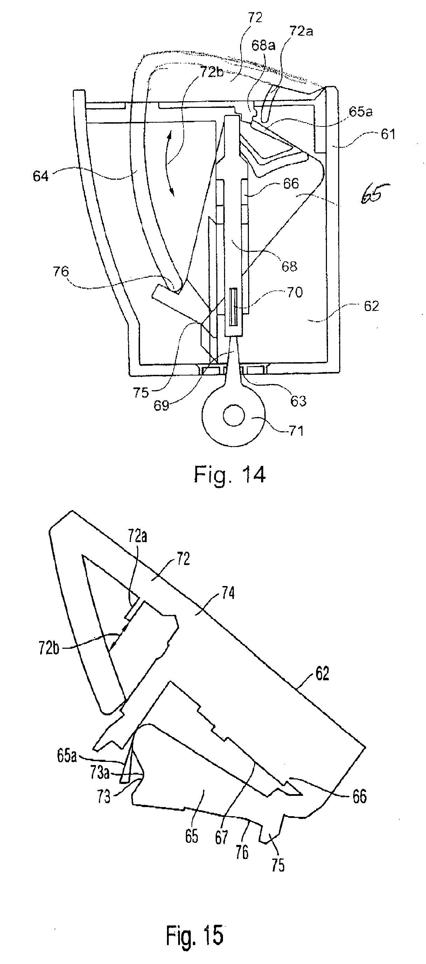

In Fig. 14 ist in einer offenen und teilweise querschnittsartigen Vorderansicht wiederum eine Stechvorrichtung gemäß einer vierten Ausführungsform der Erfindung gezeigt. Durch einen Vergleich mit einem in Fig. 15 in perspektivischer Darstellung wiedergegebenen Bauteil 62, welches innerhalb eines Basiskörpers 61 mit einer unterseitigen Öffnung 63 angeordnet ist, wird deutlich, dass wiederum ein Kunststofffederelement 64 bogenförmig ausgebildet an einem Handbetätigungselement 72 angeordnet ist, wobei in diesem Fall das Handbetätigungselement und das Kunststofffederelement 64 in einem nahezu 90°-Winkel zueinander gestellt sind.

Das Kunststofffederelement kann entlang eines Doppelpfeiles 72b gegen einen Anschlag 76 eines schwenkbaren Teiles 65 gedrückt werden, indem der Handbetätigungshebel 72 von oben nach unten gedrückt wird.

Wiederum ist Einrastmechanismus zwischen dem Bauteil 62 mit einem Vorsprung 62a und einem federartig wirkenden bzw. elastisch ausgebildeten Vorsprungsteil 65a des schwenkbaren Teiles 65 gegeben. Dieser elastische Teil 65a kann durch einen Vorsprung 72b des Handbetätigungselementes 72 ausgelöst werden, um einen Entspannungsvorgang nach stattgefundener Vorspannung des Kunststofffederelementes 64 einzuleiten.

Das Nadelhalteelement 68 weist wiederum die Nadel 70 mit einem Spitzende 69 auf.

Das schwenkbare Element 65 ist gegenüber dem restlichen Bauteil 62 an seinem unteren Ende vorzugsweise mittels eines Filmscharnieres 75 schwenkbar angeordnet.

Ebenso ist das Handbetätigungselement 72 gegenüber dem restlichen Bauteil 62 mittels eines Filmscharnieres 74 schwenkbar angeordnet.

Das Bauteil 62 ist wiederum mit einer Führungsaussparung 66, die ein unteres offenes Ende 67 aufweist, ausgestattet.

Das schwenkbare Teil 65 weist diesmal oberseitig einen V-förmigen Steuerkurvenabschnitt 73 auf, der wiederum einen tiefsten Punkt 73a beinhaltet.

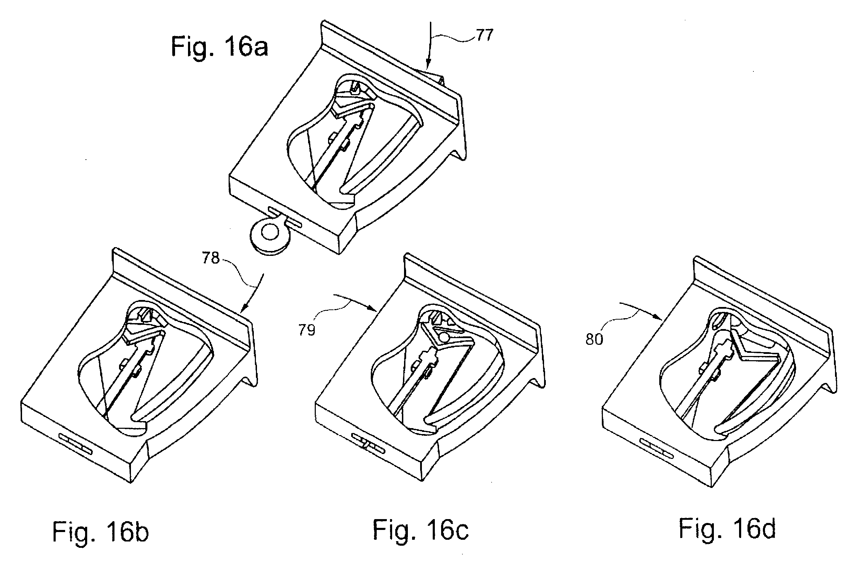

In Fig. 16a - d ist die Stechvorrichtung gemäß der vierten Ausführungsform der Erfindung in perspektivischer teilweise offener Darstellung in verschiedenen Funktionszuständen wiedergegeben. In Fig. 16a ist der Funktionszustand vor Gebrauch wiedergegeben. In diesem Zustand ist der Steuerkurvenabschnitt 73 des schwenkbaren Teiles 65 noch mit seinem rechten Ende gegenüber dem Nadelhaltelement 68 angeordnet.

Sobald eine Schwenkbewegung des Handbetätigungselementes 72 von oben nach unten stattfindet, wie es durch den Pfeil 77 wiedergegeben wird, findet eine Vorspannungsbewegung des Kunststofffederelementes 64 statt, ohne dass das schwenkbare Teil 65 verschwenkt wird, da dieses mittels des Einrastmechanismus 65a, 62a noch arretiert ist. Dies wird in Fig. 16b wiedergegeben, wobei die Schwenkbewegung durch den Pfeil 78 dargestellt wird.

Sobald der Vorsprung 72a des Handbetätigungshebels 72 auf den elastischen Vorsprung 75a des Einrastmechanismuses drückt, wird dieser aus dem Einrastanschlag 62a des Bauteiles 62 herausbewegt und somit das schwenkbare Teil 65 für eine Schwenkbewegung von links nach rechts freigegeben, wie es durch den Pfeil 79 in Fig. 16c dargestellt wird. Dies lässt das Nadelhalteelement 68 entlang dem Steuerkurvenabschnitt 73 von rechts nach links verlaufen bzw. der Steuerkurvenabschnitt 73 bewegt sich durch das Nadelhalteelement 68 von links nach rechts. Hierdurch findet ein kurzzeitiges Ausfahren und gleich anschließend wieder ein Einfahren der Nadelspitze 69 aus der unteren Öffnung 63 statt. Dies wird in Fig. 16c wiedergegeben.

In Fig. 16d wird die Stechvorrichtung nach deren Gebrauch gezeigt. In diesem Zustand ist das schwenkbare Teil 65 vollständig von links nach rechts geschwenkt worden, wie es durch den Pfeil 80 wiedergegeben ist.

Die Schwenkelemente können derart ausgebildet sein, dass sie anstatt mittels Filmscharniere an dem Basiskörper schwenkbar angeordnet sind und somit einstückig ausgebildet sind als Verschiebeelemente, welche gegenüber dem Basiskörper verschiebbar sind, ausgestaltet sind.

### Bezugszeichenliste

- 1: Basiskörper
- 2: Bauteil
- 3: Nadelausgang
- 4: Kunststofffederelement
- 5: Spannkurvenabschnitt
- 5a: Winkel
- 5b: freies Ende
- 6: Führungsaussparung
- 7: unterseitige Öffnung
- 8: Nadelhalteelement
- 9: Spitzende
- 10: Nadel/Lanzette
- 11: Schutzkappe
- 12: Handbetätigungselement
- 13: Steuerkurvenabschnitt
- 13a: Tiefpunkt des Verlaufs
- 14: Vorsprung
- 15: Schwenkbauteil
- 16: Ende
- 17: Pfeil
- 18: Verschiebebewegung
- 19: Verschiebebewegung
- 20: Verschiebebewegung
- 21: Basiskörper
- 22: Bauteil
- 24: Kunststofffederelement
- 25: Spannkurvenabschnitt
- 25a: Winkel
- 25 b: freies Ende
- 26: Führungsaussparung
- 28: Nadelhalteelement
- 29: Spitzende
- 30: Nadel
- 31: Schutzklappe
- 32: Handbetätigungselement
- 33: Steuerkurvenabschnitt
- 33a: Verlauf
- 34: Vorsprung
- 35: Schwenkteil
- 36: Unterseite
- 37: Pfeil
- 38: Verschiebebewegung
- 39: Verschiebebewegung
- 40: Verschiebebewegung
- 41: Basiskörper
- 42: Bauteil
- 42a: Einrastmechanismus/Eckkante
- 43: Öffnung
- 44: Kunststofffederelement
- 45: Schwenkelement
- 46: Führungsaussparung
- 48: Nadelhalteelement
- 48a: Haltevorsprung/Arretierungshebel
- 49: Spitzende
- 50: Nadel
- 51: Schutzklappe
- 52: Doppelpfeil
- 52a: oberes Endes des Einrastmechanismuses
- 52c: Auslösevorsprung
- 53: Steuerkurvenabschnitt
- 53a: Verlauf
- 55: Filmscharnier
- 56: Anschlag
- 57, 58, 59: Pfeil
- 59c: Vorsprung
- 60: Pfeil
- 61: Basiskörper
- 62: Bauteil
- 62a: Einrastmechanismus
- 63: unterseitige Öffnung
- 64: Kunststofffederelement
- 65: Schwenkelement
- 65a: Haltevorsprung
- 66: Führungsaussparung
- 67: offenes Ende
- 68: Nadelhalteelement
- 69: Spitzende
- 70: Nadel
- 72: Handbetätigungselement
- 72a: Auslösevorsprung
- 72b: Vorsprung
- 73: Steuerkurvenabschnitt
- 73a: Verlauf/tiefster Punkt
- 75: Filmscharnier
- 75a: elastischer Vorsprung
- 77, 78, 79, 80: Pfeil

## Patentansprüche

1. Stechvorrichtung für die Blutentnahme bei medizinischen Untersuchungen mit einem Basiskörper (1; 21; 41; 61), mindestens einer darin angeordneten und mit einem Spitzende (9; 29; 49; 69) herausfahrbaren Nadel (10; 30; 50; 70) mit einem die Nadel (10; 30; 50; 70) zumindest teilweise umfassenden Nadelhalteelement (8: 28; 48; 68) und einem Handbetätigungselement (12; 32; 52; 72) zum Auslösen einer Verschiebebewegung (19) der Nadel (10; 30; 50; 70) zusammen mit dem Nadelhalteelement (8; 28; 48; 68) gegenüber dem Basiskörper (1; 21; 41, 61), wobei in mindestens einem in dem Basiskörper (1; 21; 41; 61) angeordneten Bauteil (2; 22; 42; 62) ein verschieb- oder verschwenkbares Kunststofffederelement (4; 24; 44; 64) zur Erzeugung einer Vorspannung mittels dem Handbetätigungselement (12: 32; 52; 72), welches mit einem in dem Basiskörper (1; 21; 41; 61) verschieb- oder verschwenkbaren Steuerkurvenabschnitt (13; 33; 53; 73) verbunden ist, angeordnet ist, wobei
das Nadelhalteelement ((8; 28; 48; 68) mit dem Steuerkurvenabschnitt (13; 33; 53; 73) verbunden ist und durch Abfahren des sich verschiebenden oder verschwenkenden Steuerkurvenabschnittes (13; 33; 53; 73) selbständig in Vorwärts- und Rückwärtsrichtung (18 -20; 38 - 40) verschiebbar ist, **dadurch gekennzeichnet, dass** das Nadelhalteelement (8;28;48;68) den Steuerkurrenabschnitts (13;33;53;73) während eines Entspannungsvorganges des Kunststofffederelementes (4; 24; 44; 64) durchläuft.

2. Stechvorrichtung nach Anspruch 1,
**gekennzeichnet durch**
einen an dem Bauteil (2; 22) angeordneten, in einem Winkel (5a; 25a) zur Richtung der Verschiebebewegung (18 - 20; 38-40) des Nadelhalteelementes (8; 28) verlaufenden Spannkurvenabschnitt (5; 25), der mit dem Kunststofffederelement (4; 24), welches bogenförmig ausgebildet ist, verbunden ist und bei einer Verschiebebewegung (18) des Bauteils (2; 22) gegenüber dem Basiskörper (1; 21) in seiner Verlaufsrichtung **durch** Entlanggleiten an einem am Basiskörper (1; 21) angeordneten Vorsprung (14; 34) oder einem angeordneten nutenförmigen Element veränderbar ist.

3. Stechvorrichtung nach Anspruch 2,
**dadurch gekennzeichnet, dass**
die Verschiebebewegungen (18 - 20) des ausfahrenden Nadelhalteelementes (8) und des Bauteils (1) die gleiche Richtung aufweisen.

4. Stechvorrichtung nach Anspruch 2,
**dadurch gekennzeichnet, dass**
die Verschiebebewegungen (18 - 20) des ausfahrenden Nadelhalteelementes (8) und des Bauteils (1) senkrecht oder in einem Winkel zueinander verlaufen.

5. Stechvorrichtung nach Anspruch 2 oder 3,
**dadurch gekennzeichnet, dass**
der Vorsprung (14; 34) eine dreieckförmige Grundform aufweist.

6. Stechvorrichtung nach einem der Ansprüche 2 - 4,
**dadurch gekennzeichnet, dass**
der Spannkurvenabschnitt (5; 25) ein freies Ende (5b, 25b) aufweist, welches selbständig nach dessen Vorbeigleiten an dem Vorsprung (14; 34) in seine Ausgangslage zur Entspannung des Kunststofffederelementes (4; 24) lenkbar ist.

7. Stechvorrichtung nach einem der Ansprüche 2 - 6,
**dadurch gekennzeichnet, dass**
das Bauteil (2; 22) zusammen mit dem Steuerkurvenabschnitt (13; 33), dem Spannkurvenabschnitt (5; 25) und dem Kunststofffederelement (4; 24) einstückig ausgebildet ist.

8. Stechvorrichtung nach Anspruch 1,
**dadurch gekennzeichnet, dass**
das Kunststofffederelement (44; 64) an dem Handbetätigungselement (52; 72), welches gegenüber dem Bauteil (42; 62) schwenkbar ist (54; 74), angeordnet ist und gegen ein weiteres den Steuerkurvenabschnitt (53; 73) aufweisendes Schwenkelement (45; 65), welches gegenüber dem Bauteil (42; 62) schwenkbar gelagert ist (55; 75), vorspannbar ist.

9. Stechvorrichtung nach Anspruch 8,
**dadurch gekennzeichnet, dass**
das Handbetätigungselement (52; 72) einen Auslösevorsprung (52c; 72a) zum Verschieben eines Haltevorsprungs (48a; 65a) des Nadelhalteelements (48) oder des Schwenkelementes (65) aus einer Arretierungssteltung aufweist.

10. Stechvorrichtung nach einem der vorangegangenen Ansprüche,
**dadurch gekennzeichnet, dass**
das Handbetätigungselement (52; 72) und das Bauteil (41; 61) einen Einrastmechanismus (52a; 42a; 62a; 65a) zum Einrasten des Handbetätigungselementes (52; 72) bei einer vorbestimmbaren Stellung aufweist.

11. Stechvorrichtung nach einem der Ansprüche 8 - 10,
**dadurch gekennzeichnet, dass**
das Bauteil (42; 62) und das Schwenkelement (45; 65) einstückig ausgebildet sind.

12. Stechvorrichtung nach einem der vorangegangenen Ansprüche,
**dadurch gekennzeichnet, dass**
das Bauteil (2; 22; 42; 62) und das Handbetätigungselement (12; 32; 52; 72) einstückig ausgebildet sind.

13. Stechvorrichtung nach einem der vorangegangenen Ansprüche,
**dadurch gekennzeichnet, dass**
der Steuerkurvenabschnitt (13; 33; 53; 73) einen V-förmig identischen oder ähnlichen Verlauf (13a; 33a; 53a; 73a) aufweist.

14. Stechvorrichtung nach einem der vorangegangenen Ansprüche,
**dadurch gekennzeichnet, dass**
der Steuerkurvenabschnitt (13; 33; 53; 73) in seinem Verlauf einen Steg darstellt, der beidseitig von dem Nadelhalteelement (8; 28; 48; 68) umfasst ist, um daran in Stegverlaufsrichtung entlangzugleiten.

15. Stechvorrichtung nach einem der Ansprüche 1 -13,
**dadurch gekennzeichnet, dass**
der Steuerkurvenabschnitt (13; 33; 53; 73) und/oder der Spannkurvenabschnitt (5; 25) in seinem Verlauf eine nutenförmige Vertiefung darstellt, in welche mindestens ein auf dem Nadelhalteelement angebrachter Vorsprung eingreift, um darin in Vertiefungsverlaufsrichtung entlangzugleiten.

## Claims

1. Lancing device for taking blood for medical examinations, comprising a base body (1; 21; 41; 61), at least one needle (10; 30; 50; 70) which is arranged therein and which can be extended outwards with a tip end (9; 29; 49; 69), having a needle holding element (8; 28; 48; 68) which at least partially encompasses the needle (10; 30; 50; 70), and a manual actuating element (12; 32; 52; 72) for releasing a displacement movement (19) of the needle (10; 30; 50; 70) together with the needle holding element (8; 28; 48; 68) relative to the base body (1; 21; 41; 61), wherein a displaceable or pivotable plastic spring element (4; 24; 44; 64) for generating a pretension by means of the manual actuating element (12; 32; 52; 72), which is connected to a control curve section (13; 33; 53; 73) that can be displaced or pivoted in the base body (1; 21; 41; 61), is arranged in at least one component (2; 22; 42; 62) arranged in the base body (1; 21; 41; 61), wherein the needle holding element (8; 28; 48; 68) is connected to the control curve section (13; 33; 53; 73) and can automatically be displaced in the forward and/or backward direction (18 - 20; 38 -40) by travelling along the displaced or pivoted control curve section (13; 33; 53; 73), **characterized in that** the needle holding element (8; 28; 48; 68) passes through the control curve section (13; 33; 53; 73) during a pressure release of the plastic spring element (4; 24; 44; 64).

2. Lancing device according to claim 1, **characterized by** a tensioning curve section (5; 25) which is arranged on the component (2; 22) and runs at an angle (5a; 25a) to the direction of the displacement movement (18 - 20; 38 -40) of the needle holding element (8; 28), which tensioning curve section is connected to the plastic spring element (4; 24), of arc-shaped design, and, during a displacement movement (18) of the component (2; 22) relative to the base body (1; 21), can be varied in terms of its course direction by sliding along a protrusion (14; 34) arranged on the base body (1; 21) or along a provided groove-like element.

3. Lancing device according to claim 2 **characterized in that** the displacement movements (18 - 20) of the outwardly moving needle holding element (8) have the same direction.

4. Lancing device according to claim 2, **characterized in that** the displacement movements (18 - 20) of the outwardly moving needle holding element (8) and of the component (1) run perpendicular or at an angle to one another.

5. Lancing device according to claim 2 or 3, **characterized in that** the protrusion (14; 34) has a triangular basic shape.

6. Lancing device according to one of claims 2 to 4, **characterized in that** the tensioning curve section (5; 25) has a free end (5b; 25b) which, after sliding past the protrusion (14; 34), can automatically be guided into its starting position for the pressure release of the plastic spring element (4; 24)

7. Lancing device according to one of claims 2 to 6, **characterized in that** the component (2; 22) is formed in one piece together with the control curve section (13; 33), the tensioning curve section (5; 25) and the plastic spring element (4; 24).

8. Lancing device according to claim 1, **characterized in that** the plastic spring element (44; 64) is arranged on the manual actuating element (52; 72), which can be pivoted (54; 74) relative to the component (42; 62), and can be pretensioned against a further pivoting element (45; 65) which comprises the control curve section (53; 73) and is mounted such that it can pivot (55; 75) relative to the component (42; 62).

9. Lancing device according to claim 8, **characterized in that** the manual actuating element (52; 72) has a release protrusion (52c, 72a) for displacing a retaining protrusion (48a; 65a) of the needle holding element (48) or of the pivoting element (65) out of an arrested position.

10. Lancing device according to one of the preceding claims, **characterized in that** the manual actuating element (52; 72) and the component (41; 61) have a latching mechanism (52a; 42a; 62a; 65a) for latching the manual actuating element (52; 72) in a predefined position.

11. Lancing device according to one of claims 8 to 10, **characterized in that** the component (42; 62) and the pivoting element (45; 65) are formed in one piece.

12. Lancing device according to one of the preceding claims, **characterized in that** the component (2, 22; 42; 62) and the manual actuating element (12; 32; 52; 72) are formed in one piece.

13. Lancing device according to one of the preceding claims, **characterized in that** the control curve section (13; 33; 53; 73) has a profile (13a; 33a; 53a; 73a) identical or similar to a V-shape.

14. Lancing device according to one of the preceding claims, **characterized in that** the control curve section (13; 33; 53; 73) forms in its profile a web which is encompassed on both sides by the needle holding element (8; 28; 48; 68) so as to slide along it in the web profile direction.

15. Lancing device according to one of claims 1 to 13, **characterized in that** the control curve section (13; 33; 53; 73) and/or the tensioning curve section (5; 25) forms in its profile a groove-like depression in which there engages at least one protrusion arranged on the needle holding element, so as to slide along therein in the depression profile direction.

## Revendications

1. Dispositif de piqûre destiné au prélèvement de sang lors d'examens médicaux comprenant un corps de base (1 ; 21 ; 41 ; 61), au moins une aiguille rétractable (10 ; 30 ; 50 ; 70) agencée dans celui-ci et comprenant une extrémité pointue (9 ; 29 ; 49 ; 69) avec un élément de support de l'aiguille (8 ; 28 ; 48 ; 68) entourant au moins partiellement l'aiguille (10 ; 30 ; 50 ; 70) et un élément d'actionnement manuel (12 ; 32 ; 52 ; 72) permettant de déclencher un mouvement coulissant (19) de l'aiguille (10 ; 30 ; 50 ; 70) conjointement avec l'élément de support de l'aiguille (8 ; 28 ; 48 ; 68) par rapport au corps de base (1 ; 21 ; 41 ; 61), dans au moins un composant (2 ; 22 ; 42 ; 62) étant agencé dans le corps de base (1 ; 21 ; 41 ; 61), un élément de ressort en plastique pouvant coulisser ou pivoter (4 ; 24 ; 44 ; 64) pour produire une précontrainte au moyen de l'élément d'actionnement manuel (12 ; 32 ; 52 ; 72), lequel est relié à une section de came de commande pouvant coulisser ou pivoter (13 ; 33 ; 53 ; 73) dans le corps de base (1 ; 21 ; 41 ; 61),
l'élément de support de l'aiguille (8 ; 28 ; 48 ; 68) étant relié à la section de came de commande (13 ; 33 ; 53 ; 73) et peut coulisser de façon indépendante en sens avant et arrière (18-20 ; 38-40) par éloignement de la section de came de commande coulissante ou pivotante (13 ; 33 ; 53 ; 73),
**caractérisé en ce que** l'élément de support de l'aiguille (8 ; 28 ; 48 ; 68) traverse la section de came de commande (13 ; 33 ; 53 ; 73) pendant un processus de détente de l'élément de ressort en plastique (4 ; 24 ; 44 ; 64).

2. Dispositif de piqûre selon la revendication 1,
**caractérisé par**
une section de came de tension (5 ; 25) agencée au niveau du composant (2 ; 22), s'étendant suivant un angle (5a ; 25a) en direction du mouvement coulissant (18-20 ; 38-40) de l'élément de support de l'aiguille (8 ; 28), qui est reliée à l'élément de ressort en plastique (4 ; 24), lequel est façonné en forme d'arc, et lors d'un mouvement coulissant (18) du composant (2 ; 22) par rapport au corps de base (1 ; 21) dans sa direction d'extension, peut être modifiée sous l'effet du glissement le long d'une saillie (14 ; 34) agencée sur le corps de base (1 ; 21) ou d'un élément agencé en forme de rainure.

3. Dispositif de piqûre selon la revendication 2,
**caractérisé en ce que**
les mouvements coulissants (18-20) de l'élément de support de l'aiguille rétractable (8) et du composant (1) présentent la même direction.

4. Dispositif de piqûre selon la revendication 2,
**caractérisé en ce que**
les mouvements coulissants (18-20) de l'élément de support de l'aiguille rétractable (8) et du composant (1) s'étendent perpendiculairement ou suivant un angle l'un par rapport à l'autre.

5. Dispositif de piqûre selon la revendication 2 ou 3,
**caractérisé en ce que**
la saillie (14 ; 34) présente une forme de base triangulaire.

6. Dispositif de piqûre selon l'une quelconque des revendications 2 à 4,
**caractérisé en ce que**
la section de came de tension (5 ; 25) présente une extrémité libre (5b, 25b), laquelle après avoir glissé devant la saillie (14; 34), peut être dirigée de façon indépendante dans sa position de départ afin de détendre l'élément de ressort en plastique (4 ; 24).

7. Dispositif de piqûre selon l'une quelconque des revendications 2 à 6,
**caractérisé en ce que**
le composant (2 ; 22) est façonné d'une seule pièce avec la section de came de commande (13 ; 33), la section de came de tension (5 ; 25) et l'élément de ressort en plastique (4 ; 24).

8. Dispositif de piqûre selon la revendication 1,
**caractérisé en ce que**
l'élément de ressort en plastique (44 ; 64) est agencé au niveau de l'élément d'actionnement manuel (52 ; 72), lequel peut pivoter (54 ; 74) par rapport au composant (42 ; 62), et peut être précontraint contre un autre élément pivotant (45 ; 65) présentant la section de came de commande (53 ; 73), lequel est logé (55 ; 75) de manière pivotante par rapport au composant (42 ; 62).

9. Dispositif de piqûre selon la revendication 8,
**caractérisé en ce que**
l'élément d'actionnement manuel (52 ; 72) présente une saillie de déclenchement (52c ; 72a) pour faire coulisser une saillie d'arrêt (48a ; 65a) de l'élément de support de l'aiguille (48) ou de l'élément pivotant (65) hors d'une position d'arrêt.

10. Dispositif de piqûre selon l'une quelconque des revendications précédentes,
**caractérisé en ce que**
l'élément d'actionnement manuel (52 ; 72) et le composant (41 ; 61) présentent un mécanisme d'encliquetage (52a ; 42a ; 62a ; 65a) pour encliqueter l'élément d'actionnement manuel (52 ; 72) dans une position pouvant être prédéterminée.

11. Dispositif de piqûre selon l'une quelconque des revendications 8 à 10,
**caractérisé en ce que**
le composant (42 ; 62) et l'élément pivotant (45 ; 65) sont façonnés d'une seule pièce.

12. Dispositif de piqûre selon l'une quelconque des revendications précédentes,
**caractérisé en ce que**
le composant (2 ; 22 ; 42 ; 62) et l'élément d'actionnement manuel (12 ; 32 ; 52 ; 72) sont façonnés d'une seule pièce.

13. Dispositif de piqûre selon l'une quelconque des revendications précédentes,
**caractérisé en ce que**
la section de came de commande (13; 33; 53; 73) s'étend selon une forme identique ou similaire à une forme en V (13a ; 33a ; 53a ; 73a).

14. Dispositif de piqûre selon l'une quelconque des revendications précédentes,
**caractérisé en ce que**
la section de came de commande (13 ; 33 ; 53 ; 73) présente dans sa longueur une entretoise, qui est entourée des deux côtés par l'élément de support de l'aiguille (8 ; 28 ; 48 ; 68) afin de glisser le long de celui-ci dans la direction d'extension de l'entretoise.

15. Dispositif de piqûre selon l'une quelconque des revendications 1 à 13,
**caractérisé en ce que**
la section de came de commande (13 ; 33 ; 53 ; 73) et/ou la section de came de tension (5 ; 25) présente dans sa longueur un évidement en forme de rainure dans lequel au moins une saillie fixée sur l'élément de support de l'aiguille s'engage afin de glisser dans celui-ci dans la direction d'extension de l'évidement.
